# EUROPÄISCHE PATENTSCHRIFT

(11) **EP 1 198 453 B1**
(45) Veröffentlichungstag und Bekanntmachung des Hinweises auf die Patenterteilung: **09.04.2003**
(21) Anmeldenummer: 00949288.5
(22) Anmeldetag: 07.07.2000
(51) Int. Cl.: C07D 209/08, A61K 31/404, A61P 43/00, C07D 409/12, C07D 209/12, C07D 209/42, C07D 209/88, C07D 209/38

(54) **N-(INDOLCARBONYL-)PIPERAZINDERIVATE ALS 5-HT2A-REZEPTOR LIGANDEN**
N-(INDOLCARBONYL-)PIPERAZIN DERIVATIVES
DERIVES DE N-(INDOLCARBONYL-)PIPERAZINE

(30) Priorität: 22.07.1999 DE 19934433
(43) Veröffentlichungstag der Anmeldung: 24.04.2002
(73) Patentinhaber: MERCK PATENT GmbH, 64293 Darmstadt (DE)
(72) Erfinder: BÖTTCHER, Henning, D-64287 Darmstadt (DE); GREINER, Hartmut, D-64331 Weiterstadt (DE); HARTING, Jürgen, D-64287 Darmstadt (DE); BARTOSZYK, Gerd, D-64331 Weiterstadt (DE); SEYFRIED, Christoph, D-64342 Seeheim-Jugenheim (DE); VAN AMSTERDAM, Christoph, D-64295 Darmstadt (DE)
(86) Internationale Anmeldenummer: EP0006464
(87) Internationale Veröffentlichungsnummer: WO01007435

(56) Entgegenhaltungen:
- EP-A- 0 320 983
- WO-A-99/11641

## Beschreibung

Die Erfindung betrifft Verbindungen der Formel I 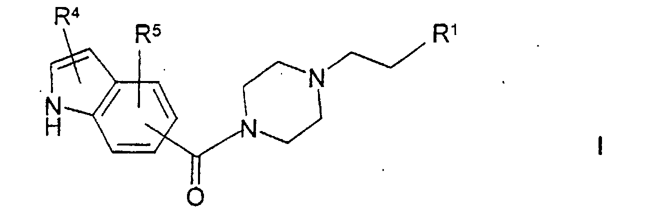 worin
- R¹: einen unsubstituierten oder durch R² und/oder R³ substituierten Phenyl- oder Naphthylrest
oder Het¹,
- R², R³: jeweils unabhängig voneinander Hal, A, OA, OH oder CN,
- R⁴, R⁵: jeweils unabhängig voneinander H, CN, Acyl, Hal, A, OA, OH, CONH₂, CONHA oder CONA₂,
- R⁴ und R⁵: zusammen auch Alkylen mit 3-5 C-Atomen,
- Het¹: ein- oder zweikerniges unsubstituiertes oder ein- oder zweifach durch Hal, A, OA oder OH substituiertes ungesättigtes heterocyclisches Ringsystem, welches eines, zwei oder drei gleiche oder verschiedene Heteroatome wie Stickstoff, Sauerstoff und Schwefel enthält,
- A: Alkyl mit 1-6 C-Atomen,
- Hal: F, Cl, Br oder I,
bedeuten,
und wobei der Indolring auch durch eine Isatineinheit ersetzt sein kann, sowie deren physiologisch unbedenklichen Salze und Solvate,
wobei (1*H*-Indol-5-yl)-(4-phenethyl-piperazin-1-yl)-methanon ausgeschlossen ist.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, dass die Verbindungen der Formel I und ihre physiologisch unbedenklichen Salze und Solvate bei guter Verträglichkeit wertvolle pharmakologische Eigenschaften besitzen, da sie Wirkungen auf das Zentralnervensystem besitzen. Die Verbindungen weisen eine starke Affinität zu 5-HT_{2A}-Rezeptoren aufweisen, weiterhin zeigen sie 5-HT_{2A}-Rezeptor-antagonistische Eigenschaften.

Zum in-vitro Nachweis der Affinität zu 5-HT_{2A}-Rezeptoren kann beispielsweise folgender Test (Beispiel A1) herangezogen werden. Die 5-HT_{2A} Rezeptoren werden sowohl [³H]Ketanserin (eine Substanz, bekannt für ihre Affinität zum Rezeptor) als auch der Testverbindung ausgesetzt. Die Abnahme der Affinität von [³H]Ketanserin zum Rezeptor ist ein Anzeichen für die Affinität der Testsubstanz zum 5-HT_{2A} Rezeptor. Der Nachweis erfolgt analog der Beschreibung von J.E. Leysen et al., Molecular Pharmacology, 1982, 21: 301-314 oder wie z.B. auch in EP 0320983 beschrieben.

Die Wirksamkeit der erfindungsgemäßen Verbindungen als 5-HT_{2A} Rezeptor-Antagonisten kann in vitro analog W. Feniuk et al., Mechanisms of 5-hydroxytryptamine-induced vasoconstriction, in: The Peripheral Actions of 5-Hydroxytryptamine, ed. Fozard JR, Oxford University Press, New York, 1989, p. 110, gemessen werden. So wird die Kontraktilität der Rattenschwanzarterie, hervorgerufen durch 5-Hydroxytryptamin, durch 5-HT_{2A} Rezeptoren vermittelt. Für das Testsystem werden Gefäßringe, präpariert aus der ventralen Rattenschwanzarterie, in einem Organbad mit einer sauerstoffgesättigten Lösung einer Perfusion unterzogen. Durch Eintrag ansteigender Konzentrationen an 5-Hydroxytryptamin in die Lösung erhält man eine Antwort auf die kumulative Konzentration an 5-HT. Danach wird die Testverbindung in geeigneten Konzentrationen in das Organbad gegeben und eine zweite Konzentrationskureve für 5-HT gemessen. Die Stärke der Testverbindung auf die Verschiebung der 5-HT induzierten Konzentrationskurve zu höheren 5-HT Konzentrationen ist ein Maß für die 5-HT_{2A}-Rezeptor-anatgonistische Eigenschaft in vitro.

Die 5-HT_{2A}-antagonistische Eigenschaft kann in vivo analog M.D.Serdar et al., Psychopharmacology, 1996, 128: 198-205, bestimmt werden.

Andere Verbindungen, die ebenfalls 5-HT₂-antagonistische Wirkungen zeigen, sind beispielweise in der EP 0320983 beschrieben.
Ähnliche Piperazinderivate mit antiarrhythmischen Eigenschaften sind z.B. in der EP 0431945 offenbart. Andere Indolcarbonylderivate mit analgetischen Eigenschaften sind in der EP 0599240 beschrieben. In der WO 99/11641 sind Phenylindolderivate mit 5-HT₂-antagonistischen Eigenschaften beschrieben.

Die Verbindungen der Formel I eignen sich daher sowohl in der Veterinärais auch in der Humanmedizin zur Behandlung von Funktionsstörungen des Zentralnervensystems sowie von Entzündungen. Sie können zur Prophylaxe und zur Bekämpfung der Folgen cerebraler Infarktgeschehen (apoplexia cerebri) wie Schlaganfall und cerebraler Ischämien sowie zur Behandlung extrapyramidal-motorischer Nebenwirkungen von Neuroleptika sowie des Morbus Parkinson, zur akuten und symptomatischen Therapie der Alzheimer Krankheit sowie zur Behandlung der amyotrophen Lateralsklerose verwendet werden. Ebenso eignen sie sich als Therapeutika zur Behandlung von Hirn- und Rückenmarkstraumata. Insbesondere sind sie jedoch geeignet als Arzneimittelwirkstoffe für Anxiolytika, Antidepressiva, Antipsychotika, Neuroleptika, Antihypertonika und/oder zur positiven Beeinflussung von Zwangsverhalten (obsessive-compulsive disorder, OCD; z.B. WO 9524194), Angstzuständen sowie physiologischen Veränderungen, die mit Angstzuständen einhergehen wie z.B. Tachycardie, Tremor oder Schwitzen (z.B. EP 319962), Panikattacken, Psychosen, Schizophrenie, Anorexie, wahnhaften Zwangsvorstellungen, Agoraphobie, Migräne, der Alzheimer Krankheit, Schlafstörungen wie auch Schlafapnoe, tardiver Dyskinesien, Lernstörungen, altersabhängiger Erinnerungsstörungen, Essstörungen wie Bulimie, Drogenmissbrauch wie z.B. von Alkohol, Opiaten, Nikotin, Psychostimulantien wie z.B. Kokain oder Amphetaminen (z.B. US 6004980), Sexualfunktionsstörungen, Schmerzuzuständen aller Art und Fibromyalgie (z.B. WO 9946245).
Die Verbindungen der Formel I eignen sich zur Behandlung extrapyramidaler Nebenwirkungen (extrapyramidal side effects EPS) bei der neuroleptischen Drogentherapie. EPS ist gekennzeichnet durch Parkinson-ähnliche Syndrome, Akathisie und dystonische Reaktionen (z.B. EP 337136). Weiter sind sie geeignet zur Behandlung der nervösen Anorexie, Angina, Reynaud's Phänomen, koronaren Vasospasmen, bei der Prophylaxe von Migräne (z.B. EP 208235), Schmerz und Neuralgien (z.B. EP 320983), zur Behandlung des Rett-Syndroms mit autistischen Charakterzügen, des Asperger-Syndroms, des Autismus und autistischen Störungen, bei Konzentrationsmangelzuständen, Entwicklungsstörungen, Hyperaktivitätszuständen mit mentaler Unterentwicklung und stereotypen Verhaltenszuständen (z.B. WO 9524194).

Desweiteren sind sie geeignet zur Behandlung von endokrinen Erkrankungen wie Hyperprolactinaemie, ferner bei Vasospasmen, thrombotischen Erkrankungen (z.B. WO 9946245), Hypertension und gastrointestinaien Erkrankungen.
Ferner sind sie geeignet zur Behandlung cardiovaskulärer Erkrankungen sowie extrapyramidaler Symptome wie in der WO 99/11641 auf Seite 2, Zeile 24-30 beschrieben.
Die erfindungsgemäßen Verbindungen eignen sich weiter zur Verminderung des Augeninnendruckes und zur Glaucombehandlung.
Sie sind auch zur Prophylaxe und Behandlung von Vergiftungserscheinungen bei der Gabe von Ergovalin bei Tieren geeignet.
Die Verbindungen eignen sich weiterhin zur Behandlung von Erkrankungen des Herz-Kreislaufsystems (WO 99/11641, Seite 3, Zeile 14-15). Die erfindungsgemäßen Verbindungen können auch zusammen mit anderen Wirkstoffen in der Behandlung der Schizophrenie eingesetzt werden. Als andere Wirkstoffe kommen die in der WO 99/11641 auf Seite 13, Zeile 20-26 genannten Verbindungen in Frage.

Ferner können sie als Zwischenprodukte zur Herstellung weiterer Arzneimittelwirkstoffe eingesetzt werden.

Gegenstand der Erfindung sind die N-(Indolcarbonyl-)piperazinderivate der Formel I sowie ihre physiologisch unbedenklichen Säureadditionssalze. Gegenstand der Erfindung sind auch die Solvate, z.B. Hydrate oder Alkoholate, dieser Verbindungen.

Gegenstand der Erfindung sind dementsprechend die Verbindungen der Formel I sowie ein Verfahren zur Herstellung von Verbindungen der Formel I gemäß Anspruch 1.

Das Verfahren zur Herstellung von Verbindungen der Formel I gemäß Anspruch 1, wobei (1*H*-Indol-5-yl)-(4-phenethyl-piperazin-1-yl)-methanon ausgeschlossen ist, ist dadurch gekennzeichnet, daß man
a) eine Verbindung der Formel II 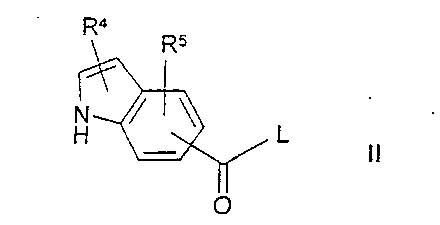 worin L Cl, Br, I oder eine freie oder reaktionsfähig funktionell abgewandelte OH-Gruppe bedeutet,
   und R⁴ und R⁵ die in Anspruch 1 angegebenen Bedeutungen haben, mit einer Verbindung der Formel III 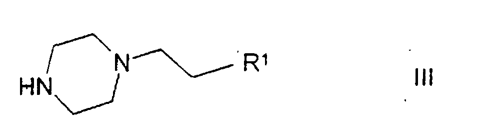 worin R¹ die in Anspruch 1 angegebene Bedeutung hat,
   umsetzt,
   oder
b) eine Verbindung der Formel IV 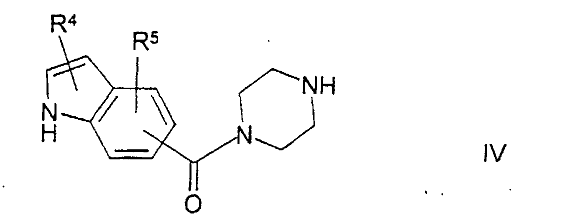 worin R⁴ und R⁵ die in Anspruch 1 angegebenen Bedeutungen haben, mit einer Verbindung der Formel V

   L-CH₂-CH₂-R¹ V

   worin L Cl, Br, I oder eine freie oder reaktionsfähig funktionell abgewandelte OH-Gruppe bedeutet, und R¹ die in Anspruch 1 angegebene Bedeutung hat,
   umsetzt,
   oder
c) gegebenenfalls einen der Reste R¹, R⁴ und/oder R⁵ in einen anderen Rest R¹, R⁴ und/oder R⁵ umwandelt, indem man beispielsweise eine OA-Gruppe unter Bildung einer OH-Gruppe spaltet und/oder eine CHO-Gruppe in eine CN-Gruppe umwandelt,
   und/oder eine erhaltene Base der Formel I durch Behandeln mit einer Säure in eines ihrer Salze umwandelt.

Gegenstand der Erfindung sind auch die Verbindungen der Formel I gemäß Anspruch 1, sowie ihrer physiologisch unbedenklichen Salze und Solvate, wobei (1H-Indol-5-yl)-(4-phenethyl-piperazin-1-yl)-methanon ausgeschlossen ist, als Arzneimittel.

Gegenstand der Erfindung sind insbesondere die Verbindungen der Formel I gemäß Anspruch 1, sowie ihrer physiologisch unbedenklichen Salze und Solvate als Arzneimittel mit 5-HT_{2A}-Rezeptor-antagonistischer Wirkung.

Gegenstand der Erfindung sind auch die Verbindungen der Formel I sowie deren Enantiomere sowie Diastereomere und deren Salze.

Der Indolring kann auch durch eine Isatineinheit ersetzt sein. isatin ist ein in 2- und 3-Stellung durch Oxo substituiertes Indol = Indol-2,3-dion.

Für alle Reste, die mehrfach auftreten, wie z.B. A oder Hal, gilt, daß deren Bedeutungen unabhängig voneinander sind.

Der Rest A bedeutet Alkyl und hat 1 bis 6 , vorzugsweise 1, 2, 3 oder 4, insbesondere 1 oder 2 C-Atome. Alkyl bedeutet daher insbesondere z.B. Methyl, weiterhin Ethyl, n-Propyl, Isopropyl, n-Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, ferner Trifluormethyl oder Pentafluorethyl.

Acyl hat vorzugsweise 1-6 C-Atome und bedeutet z.B. Formyl, Acetyl, Propionyl, Butyryl, ferner Trifluoracetyl oder Pentafluorpropionyl.

Alkylen ist Propylen, Butylen oder Pentylen.
OA ist vorzugsweise Methoxy, ferner auch Ethoxy, n-Propoxy, Isopropoxy, n-Butoxy, Isobutoxy, sek.-Butoxy oder tert.-Butoxy.

Hal bedeutet Fluor, Chlor, Brom oder Iod, insbesondere Fluor oder Chlor.

R¹ ist unsubstituiertes, vorzugsweise - wie angegeben - monosubstituiertes Phenyl oder Naphthyl, im einzelnen bevorzugt Phenyl, o-, m- oder p-Tolyl, o-, m- oder p-Ethylphenyl, o-, m- oder p-Propylphenyl, o-, m- oder p-Isopropylphenyl, o-, m- oder p-tert.-Butylphenyl, o-, m- oder p-Trifluormethylphenyl, o-, m- oder p-Hydroxyphenyl, o-, m- oder p-Nitrophenyl, o-, m- oder p-(Trifluormethoxy)-phenyl, o-, m- oder p-Cyanphenyl, o-, m- oder p-Methoxyphenyl, o-, m- oder p-Ethoxyphenyl, o-, m- oder p-Fluorphenyl, o-, m- oder p-Bromphenyl, o-, m- oder p- Chlorphenyl, o-, m- oder p-(Difluormethoxy)-phenyl, o-, m- oder p-(Fluormethoxy)-phenyl, weiter bevorzugt 2,3-, 2,4-, 2,5-, 2,6-, 3,4- oder 3,5-Difluorphenyl, 2,3-, 2,4-, 2,5-, 2,6-, 3,4- oder 3,5-Dichlorphenyl, 2,3-, 2,4-, 2,5-, 2,6-, 3,4- oder 3,5-Dibromphenyl, 2-Chlor-3-methyl-, 2-Chlor-4-methyl-, 2-Chlor-5-methyl-, 2-Chlor-6-methyl-, 2-Methyl-3-chlor-, 2-Methyl-4-chlor-, 2-Methyl-5-chlor-, 2-Methyl-6-chlor-, 3-Chlor-4-methyl-, 3-Chior-5-methyl- oder 3-Methyl-4-chlorphenyl, 2-Brom-3-methyl-, 2-Brom-4-methyl-, 2-Brom-5-methyl-, 2-Brom-6-methyl-, 2-Methyl-3-brom-, 2-Methyl-4-brom-, 2-Methyl-5-brom-, 2-Methyl-6-brom-, 3-Brom-4-methyl-, 3-Brom-5-methyl- oder 3-Methyl-4-bromphenyl, 2,4- oder 2,5-Dinitrophenyl, 2,5- oder 3,4-Dimethoxyphenyl, 3-Nitro-4-chlorphenyl, 2,3,4-, 2,3,5-, 2,3,6-, 2,4,6- oder 3,4,5-Trichlorphenyl, 2,4,6-tri-tert.-Butylphenyl, ferner bevorzugt 2-Nitro-4-(trifluormethyl)phenyl, 3,5-Di-(trifluormethyl)-phenyl, 2,5-Dimethylphenyl, 2-Hydroxy-3,5-dichlorphenyl, 2-Fluor-5- oder 4-Fluor-3-(trifluormethyl)-phenyl, 4-Chlor-2- oder 4-Chlor-3-(trifluormethyl)-, 2-Chlor-4- oder 2-Chlor-5-(trifluormethyl)-phenyl, 4-Brom-2- oder 4-Brom-3-(trifluormethyl)-phenyl, p-Iodphenyl, 2-Nitro-4-methoxyphenyl, 2,5-Dimethoxy-4-nitrophenyl, 2-Methyl-5-nitrophenyl, 2,4-Dimethyl-3-nitrophenyl, 4-Fluor-3-chlorphenyl, 4-Fluor-3,5-dimethylphenyl, 2-Fluor-4-Bromphenyl, 2,5-Difluor-4-bromphenyl, 2,4-Dichlor-5-methylphenyl, 3-Brom-6-methoxyphenyl, 3-Chlor-6-methoxyphenyl, 2-Methoxy-5-methylphenyl oder 2,4,6-Triisopropylphenyl.

R¹ ist auch Het¹.
Het¹ ist vorzugsweise 2- oder 3-Furyl, 2- oder 3-Thienyl, 1-, 2- oder 3-Pyrrolyl, 1-, 2, 4- oder 5-Imidazolyl, 1-, 3-, 4- oder 5-Pyrazolyl, 2-, 4- oder 5-Oxazolyl, 3-, 4- oder 5-Isoxazolyl, 2-, 4- oder 5-Thiazolyl, 3-, 4- oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3- oder 5-yl, 1-oder 5-Tetrazolyl, 1,2,3-Oxadiazol-4- oder -5-yl, 1,2,4-Oxadiazol-3- oder- 5-yl, 1,3,4-Thiadiazol-2- oder -5-yl, 1,2,4-Thiadiazol-3- oder -5-yl, 1,2,3-Thiadiazol-4- oder -5-yl, 2-, 3-, 4-, 5- oder 6-2H-Thiopyranyl, 2-, 3- oder 4-4-H-Thiopyranyl, 3- oder 4-Pyridazinyl, Pyrazinyl, 2-, 3-, 4-, 5- 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 4- oder 5-Benzimidazolyl, 1-, 3-, 4-, 5-, 6- oder 7-Benzopyrazoiyl, 2-, 4-, 5-, 6- oder 7-Benzoxazolyl, 3-, 4-, 5-, 6- oder 7- Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2,1,3-oxadiazolyl, 2-, 3-, 4-, 5-, 6-, 7- oder 8-Chinolyl, 1-, 3-, 4-, 5-, 6-, 7- oder 8-Isochinolyl, 3-, 4-, 5-, 6-, 7- oder 8-Cinnolinyl, 2-, 4-, 5-, 6-, 7- oder 8-Chinazolinyl.

R¹ bedeutet ganz besonders bevorzugt Phenyl, p-Chlorphenyl, p-Fluorphenyl, Thiophen-2-yl, 5-Chlor-thiophen-2-yl, 2,5-Dichlor-thiophen-3-yl und 2- oder 3-Furyl.

R⁴, R⁵ bedeuten jeweils unabhängig voneinander vorzugsweise H, Hal, Alkyl mit 1-6 C-Atomen, Alkoxy mit 1-6 C-Atomen oder Hydroxy, ferner Cyan oder Acyl.

R⁴ bedeutet vorzugsweise H, Hai, A, OA, OH, CN oder Acyl. R⁵ bedeutet vorzugsweise H.

Bevorzugt sind solche Verbindungen der Formel I, in denen der R¹-CH₂-CH₂-piperazin-carbonyl-Rest die 4-, 5-, 6- oder 7-Stellung des Indolrings substituiert.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln la bis li ausgedrückt werden, die der Formel I entsprechen und worin die nicht näher bezeichneten Reste die bei der Formel I angegebene Bedeutung haben, worin jedoch
- in la R¹: Phenyl bedeutet;
- in Ib R¹: unsubstituiertes oder einfach durch Hal substituiertes Phenyl bedeutet;
- in Ic R¹: einfach durch Hal substituiertes Phenyl oder Het¹ bedeutet;
- in Id R¹: unsubstituiertes oder einfach durch Hal substituiertes Phenyl oder Het¹
bedeutet;
- in le R¹: unsubstituiertes oder einfach durch Hal substituiertes Phenyl oder Het¹;
- Het¹: unsubstituiertes oder ein- oder zweifach durch Hal oder A substituiertes ungesättigtes heterocyclisches Ringsystem, welches eines oder zwei gleiche oder verschiedene Heteroatome wie Stickstoff, Sauerstoff und Schwefel enthält,
bedeutet;
- in If R¹: unsubstituiertes oder einfach durch Hal substituiertes Phenyl oder Het¹,
- R⁴, R⁵: jeweils unabhängig voneinander H, Hal oder A,
- Het¹: unsubstituiertes oder ein- oder zweifach durch Hal oder A substituiertes ungesättigtes heterocyclisches Ringsystem, welches eines oder zwei gleiche oder verschiedene Heteroatome wie Stickstoff, Sauerstoff und Schwefel enthält,
bedeutet;
- in Ig R¹: unsubstituiertes oder einfach durch Hal substituiertes Phenyl oder Het¹,
- R⁴, R⁵: jeweils unabhängig voneinander H, Hal oder A,
- R⁴ und R⁵: zusammen auch Alkylen mit 3-5 C-Atomen,
- Het¹: unsubstituiertes oder ein- oder zweifach durch Hal oder A substituiertes Thienyl oder Furyl,
bedeutet;
- in Ih R¹: unsubstituiertes oder einfach durch Hal substituiertes Phenyl oder Het¹,
- R⁴: H, Hal, CN, Acyl oder A,
- R⁵: H,
- R⁴ und R⁵: zusammen auch Alkyien mit 3-5 C-Atomen,
- Het¹: unsubstituiertes oder ein- oder zweifach durch Hal oder A substituiertes Thienyl oder Furyl,
bedeutet;
- in li R¹: unsubstituiertes oder einfach durch Hal substituiertes Phenyl oder Naphthyl
oder Het¹,
- R⁴: H, Hal, CN, Acyl, A oder CONH₂,
- R⁵: H,
- R⁴ und R⁵: zusammen auch Alkylen mit 3-5 C-Atomen,
- Het¹: unsubstituiertes oder ein- oder zweifach durch Hal oder A substituiertes Thienyl oder Furyl,
bedeutet,
und wobei der Indolring auch durch eine Isatineinheit ersetzt sein kann.

Die Verbindungen der Formel I und auch die Ausgangsstoffe zu ihrer Herstellung werden im übrigen nach an sich bekannten Methoden hergestellt, wie sie in der Literatur (z.B. in Standardwerken wie Houben-Weyl, Methoden der Organischen Chemie, Georg Thieme Verlag, Stuttgart; Organic Reactions, John Wiley & Sons, Inc., New York;) beschrieben sind, und zwar unter Reaktionsbedingungen, wie sie für die genannten Umsetzungen bekannt und geeignet sind. Dabei kann man auch von an sich bekannten, hier nicht näher erwähnten Varianten Gebrauch machen.

Die Ausgangsstoffe für das beanspruchte Verfahren können gewünschtenfalls auch in situ gebildet werden, derart, dass man sie aus dem Reaktionsgemisch nicht isoliert, sondern sofort weiter zu den Verbindungen der Formel I umsetzt. Andererseits ist es möglich, die Reaktion stufenweise durchzuführen.

In den Verbindungen der Formel II und V ist der Rest L vorzugsweise Cl oder Br; er kann jedoch auch I, OH oder auch bevorzugt eine reaktionsfähig funktionell abgewandelte OH-Gruppe bedeuten, insbesondere Alkylsulfonyloxy mit 1-6 (z.B. Methansulfonyloxy) oder Arylsulfonyloxy mit 6-10 C-Atomen (z.B. Benzolsuifonyloxy, p-Toluoisulfonyloxy, 1- oder 2-Naphthalinsulfonyloxy) oder auch Trichlormethoxy, Alkoxy, wie z.B. Methoxy, Ethoxy, Propoxy oder Butoxy, ferner auch Phenoxy.

Die Verbindungen der Formel I können vorzugsweise erhalten werden, indem man Verbindungen der Formel II mit Verbindungen der Formel III umsetzt.
Die Ausgangsstoffe der Formeln II und III sind in der Regel bekannt; die nicht bekannten Verbindungen der Formeln II und III können leicht analog zu den bekannten Verbindungen hergestellt werden.

Die Umsetzung der Verbindungen II und III verläuft nach Methoden, wie sie für die Alkylierung bzw. Acylierung von Aminen aus der Literatur bekannt sind. Es ist aber auch möglich, die Verbindungen in Gegenwart eines indifferenten Lösungsmittels umzusetzen. Als Lösungsmittel eignen sich z.B. Kohlenwasserstoffe, wie Benzol, Toluol, Xylol; Ketone wie Aceton, Butanon; Alkohole wie Methanol, Ethanol, Isopropanol, N-Butanol; Ether wie Tetrahydrofuran (THF) oder Dioxan; Amide wie Dimethylformamid (DMF) oder N-Methyl-pyrrolidon; Nitrile wie Acetonitril, gegebenenfalls auch Gemische dieser Lösungsmittel untereinander oder Gemische mit Wasser. Der Zusatz eines säurebindenden Mittels, beispielsweise eines Alkali- oder Erdalkalimetallhydroxids, -carbonats oder -bicarbonats oder eines anderen Salzes einer schwachen Säure der Alkali- oder Erdalkalimetalle, vorzugsweise des Kaliums, Natriums oder Calciums, oder der Zusatz einer organischen Base wie Triethylamin, Dimethylanilin, Pyridin oder Chinolin oder eines Überschusses Piperazin-Derivates der Formel II kann günstig sein. Die Reaktionszeit liegt je nach den angewendeten Bedingungen zwischen einigen Minuten und 14 Tagen, die Reaktionstemperatur zwischen etwa 0 und 150°, normalerweise zwischen 20 und 130°.

Desweiteren kann man Verbindungen der Formel I herstellen, indem man Amine der Formel IV mit einer Komponte der Formel V enthaltend den Rest R¹ umsetzt.
Die jeweiligen Komponenten sind in der Regel bekannt oder können wie schon beschrieben nach bekannten Verfahren hergestellt werden.

Eine erhaltene Base der Formel I kann mit einer Säure in das zugehörige Säureadditionssalz übergeführt werden. Für diese Umsetzung eignen sich Säuren, die physiologisch unbedenkliche Salze liefern. So können anorganische Säuren verwendet werden, z.B. Schwefelsäure, Halogenwasserstoffsäuren wie Chlorwasserstoffsäure oder Bromwasserstoffsäure, Phosphorsäuren wie Orthophosphorsäure, Salpetersäure, Sulfaminsäure, ferner organische Säuren, im einzelnen aliphatische, alicyclische, araliphatische, aromatische oder heterocyclische ein- oder mehrbasige Carbon-, Sulfon- oder Schwefelsäuren, wie Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Benzoesäure, Salicylsäure, 2-Phenylpropionsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure; Benzolsulfonsäure, p-Toluolsulfonsäure, Naphthalin-monound -disulfonsäuren, Laurylschwefelsäure.

Die freien Basen der Formel I können, falls gewünscht, aus ihren Salzen durch Behandlung mit starken Basen wie Natrium- oder Kaliumhydroxid, Natrium- oder Kaliumcarbonat in Freiheit gesetzt werden, sofern keine weiteren aciden Gruppen im Molekül vorliegen. In jenen Fällen, wo die Verbindungen der Formel I über freie Säuregruppen verfügen, kann durch Behandlung mit Basen ebenfalls eine Salzbildung erreicht werden. Als Basen eignen sich Alkalimetallhydroxide, Erdalkalimetalihydroxide oder organische Basen in Form von primären, sekundären oder tertiären Aminen.

Gegenstand der Erfindung sind weiterhin die erfindungsgemäßen Arzneimittel mit 5-HT_{2A}-Rezeptor-antagonistischer Wirkung zur Behandlung von Psychosen, Schizophrenie, Depression, neurologischen Störungen, Gedächtnisstörungen, Morbus Parkinson, amyotropher Lateralsklerose, der Alzheimer Krankheit, der Huntington Krankheit, Essstörungen wie Bulimie, nervöser Anorexie, des prämenstrualen Syndroms und/oder zur positiven Beeinflussung von Zwangsverhalten (obsessive-compulsive disorder, OCD).

Gegenstand der Erfindung ist auch eine pharmazeutische Zubereitung, enthaltend mindestens ein erfindungsgemäßes Arzneimittel sowie gegebenenfalls Träger- und/oder Hiifsstoffe und gegebenenfalls andere Wirkstoffe.
Hierbei können die Arzneimittel zusammen mit mindestens einem festen, flüssigen und/oder halbflüssigen Träger- oder Hilfsstoff und gegebenenfalls in Kombination mit einem oder mehreren weiteren Wirkstoff(en) in eine geeignete Dosierungsform gebracht werden.

Gegenstand der Erfindung ist weiterhin die Verwendung der erfindungsgemäßen Verbindungen und/oder von deren physiologisch unbedenklichen Salzen und Solvaten zur Herstellung eines Arzneimittels mit 5-HT_{2A}-Rezeptor-antagonistischer Wirkung.

Gegenstand der Erfindung ist auch die Verwendung der erfindungsgemäßen Verbindungen und/oder von deren physiologisch unbedenklichen Salzen und Solvaten zur Herstellung eines Arzneimittels mit 5-HT_{2A}-Rezeptor-antagonistischer Wirkung zur Behandlung von Psychosen, Schizophrenie, Depression, neurologischen Störungen, Gedächtnisstörungen, Morbus Parkinson, amyotropher Lateralsklerose, der Alzheimer Krankheit, der Huntington Krankheit, Essstörungen wie Bulimie, nervöser Anorexie, des prämenstrualen Syndroms und/oder zur positiven Beeinflussung von Zwangsverhalten (obsessive-compulsive disorder, OCD).

Die pharmazeutischen Zubereitungen können als Arzneimittel in der Human- und Veterinärmedizin eingesetzt werden. Als Trägersubstanzen kommen organische oder anorganische Stoffe in Frage, die sich für die enterale (z.B. orale), parenterale oder topische Applikation eignen und mit den neuen Verbindungen nicht reagieren, beispielsweise Wasser, pflanzliche Öle, Benzylalkohole, Polyethylenglykole, Gelatine, Kohlehydrate wie Lactose oder Stärke, Magnesiumstearat, Talk, Vaseline. Zur enteralen Applikation dienen insbesondere Tabletten, Dragees, Kapseln, Sirupe, Säfte, Tropfen oder Suppositoren, zur parenteralen Applikation Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenden Lyophilisate z.B. zur Herstellung von Injektionspräparaten verwendet werden.

Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder Aromastoffe enthalten. Sie können, falls erwünscht, auch einen oder mehrere weitere Wirkstoffe enthalten, z.B, ein oder mehrere Vitamine.

Dabei werden die erfindungsgemäßen Substanzen in der Regel in Analogie zu bekannten Präparaten verabreicht, vorzugsweise in Dosierungen zwischen etwa 0,1 und 500 mg, insbesondere zwischen 5 und 300 mg pro Dosierungseinheit. Die tägliche Dosierung liegt vorzugsweise zwischen etwa 0,01 und 250 mg/kg, insbesondere zwischen 0,02 und 100 mg/kg Körpergewicht.

Dabei werden die erfindungsgemäßen Substanzen in der Regel vorzugsweise in Dosierungen zwischen etwa 1 und 500 mg, insbesondere zwischen 5 und 100 mg pro Dosierungseinheit verabreicht. Die tägliche Dosierung liegt vorzugsweise zwischen etwa 0,02 und 10 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachstehenden Beispielen bedeutet "übliche Aufarbeitung": Man entfernt, falls erforderlich, das Lösungsmittel, gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, filtriert, engt ein und reinigt durch Chromatographie an Kieselgel und/oder durch Kristallisation.

### Beispiel A1

Herstellung einer Suspension von 5-HT_{2A} Rezeptoren:
Frontaler Rattencortex wird in eiskaltem Puffer homogenisiert. Das Homogenat wird 10 Minuten bei 4°C und 50000 X zentrifugiert. Das Pellet wird in 2,5 ml eiskaltem Trispuffer resuspendiert, mit 10 ml zusätzlichem Puffer aufgefüllt und wie beschrieben zentrifugiert. Danach wird das Pellet in Puffer resuspendiert und zu einem Homogenat verdünnt, das 60 mg Material/ml enthält.
In die Inkubationsröhrchen werden 0,1 ml der Suspension, 100 µl einer 5 nM Lösung von [³H]Ketanserin, 100 µl einer Lösung der Testverbindung (Konzentration im Bereich von 10⁻⁵ bis 10⁻¹⁰ Mol pro Liter) gegeben und mit Puffer auf 1 ml aufgefüllt. Die Röhrchen werden 15 Minuten bei 37 °C inkubiert. Nach Abbrechen der Inkubation durch Eintauchen der Röhrchen in ein Eisbad wird die gekühlte Suspension durch ein Glasfilter unter Vakuum filtriert. Die Filter werden 3x mit 5 ml kaltem Puffer gewaschen und dann in Szintillationsröhrchen überführt. Die Filter werden mittels Flüssigszintillations-Spektrometrie in 8 ml Triton-X-Szintillatorflüssigkeit analysiert.

### Beispiel 1

Eine Lösung von 2,0 g 4-Carboxyindol und 8,1 g 2-Chlor-1-methylpyridiniumjodid in 60 ml N-Methylpyrrolidon (NMP) wird mit einer Lösung von 2,36 g 4-Phenethyl-piperazin und 8,2 g Ethyl-diisopropylamin (EDIPA) in 20 ml NMP versetzt und 3 Stunden bei Raumtemperatur nachgerührt. Man arbeitet wie üblich auf und erhält das Rohprodukt. Dieses wird in Aceton gelöst und mit wässeriger Salzsäure wird das Hydrochlorid ausgefällt. Nach Trocknung erhält man 4,59 g (1*H*-Indol-4-yl)-(4-phenethylpiperazin-1-yl)-methanon, Hydrochlorid

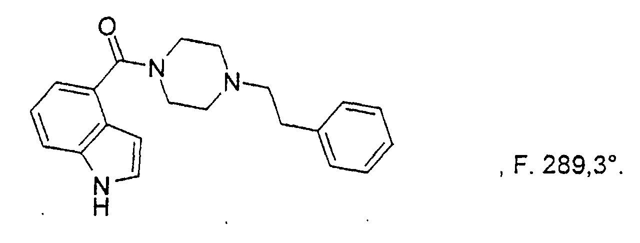

Analog erhält man die nachstehenden Verbindungen
(1*H*-Indol-4-yl)-[4-(4-fluorphenethyl)-piperazin-1-yl]-methanon, Hydrochlorid, F. 250°;
(1*H*-Indol-4-yl)-[4-(thiophen-2-ylethyl)-piperazin-1-yl]-methanon,
(1*H*-Indol-4-yl)-[4-(5-chlor-thiophen-2-ylethyl)-piperazin-1-yl]-methanon,
(1*H*-Indol-4-yl)-[4-(thiophen-3-ylethyl)-piperazin-1-yl]-methanon,
(1*H*-Indol-4-yl)-[4-(2,5-dichlor-thiophen-3-ylethyl)-piperazin-1-yl]-methanon, Hydrochlorid, F. 166-168°;

(1*H*-Indol-5-yl)-[4-(4-fluorphenethyl)-piperazin-1-yl]-methanon,
(1*H*-Indol-5-yl)-[4-(thiophen-2-ylethyl)-piperazin-1-yl]-methanon,
(1*H*-Indol-5-yl)-[4-(5-chlor-thiophen-2-ylethyl)-piperazin-1-yl]-methanon,
(1*H*-Indol-5-yl)-[4-(thiophen-3-ylethyl)-piperazin-1-yl]-methanon,
(1*H*-lndol-5-yl)-[4-(2,5-dichlor-thiophen-3-ylethyl)-piperazin-1-yl]-methanon,
(3-Formyl-1*H*-indol-5-yl)-[4-(4-fluorphenethyl)-piperazin-1-yl]-methanon, Hydrochlorid, F. 240,9°;

(1*H*-Indol-6-yl)-(4-phenethyl-piperazin-1-yl)-methanon,
(1*H*-Indol-6-yl)-[4-(4-fluorphenethyl)-piperazin-1-yl]-methanon, Hydrochlorid, F. 284,0-284,4°;
(1*H*-Indol-6-yl)-[4-(thiophen-2-ylethyl)-piperazin-1-yl]-methanon, Hydrochlorid, F. 204,2-205,7°;
(1*H*-Indol-6-yl)-[4-(5-chlor-thiophen-2-ylethyl)-piperazin-1-yl]-methanon, Hydrochlorid, F. 251,0-252,5°;
(1*H*-Indol-6-yl)-[4-(thiophen-3-ylethyl)-piperazin-1-yl]-methanon,
(1*H*-Indol-6-yl)-[4-(2,5-dichlor-thiophen-3-ylethyl)-piperazin-1-yl)-methanon, Hydrochlorid, F. 240-241°;
(3-Formyl-1*H*-indol-6-yl)-[4-(4-fluorphenethyl)-piperazin-1-yl]-methanon,
(3-Cyan-1*H*-indol-6-yl)-[4-(4-fluorphenethyl)-piperazin-1-yl]-methanon, Hydrochlorid, F. 280°;

(1*H*-Indol-7-yl)-(4-phenethyl-piperazin-1-yl)-methanon, Hydrochlorid, F. 221°;
(1*H*-Indol-7-yl)-[4-(4-fluorphenethyl)-piperazin-1-yl]-methanon, Hydrochlorid, F. 274°;
(1*H*-Indol-7-yl)-[4-(thiophen-2-ylethyl)-piperazin-1-yl]-methanon,
(1*H*-Indol-7-yl)-[4-(5-chlor-thiophen-2-ylethyl)-piperazin-1-yl]-methanon, Hydrochlorid, F. 251,0-252,5°;
(1*H*-Indol-7-yl)-[4-(thiophen-3-ylethyl)-piperazin-1-yl]-methanon,
(1*H*-Indol-7-yl)-[4-(2,5-dichlor-thiophen-3-ylethyl)-piperazin-1-yl]-methanon,
(3-Formyl-1*H*-indol-7-yl)-[4-(4-fluorphenethyl)-piperazin-1-yl]-methanon, Hydrochlorid, F. 287°;
(3-Cyan-1*H*-indol-7-yl)-[4-(4-fluorphenethyl)-piperazin-1-yl]-methanon, Hydrochlorid, F. >300°;

(2,3-Dimethyl-1*H*-indol-7-yl)-(4-phenethyl-piperazin-1-yl)-methanon,
(2,3-Dimethyl-1*H*-indol-7-yl)-[4-(4-fluorphenethyl)-piperazin-1-yl]-methanon, 86,5-89°;
(2,3-Dimethyl-1*H*-indol-7-yl)-[4-(thiophen-2-ylethyl)-piperazin-1-yl]-methanon,
(2,3-Dimethyl-1*H*-indol-7-yl)-[4-(5-chlor-thiophen-2-ylethyl)-piperazin-1-yl]-methanon,
(2,3-Dimethyl-1*H*-indol-7-yl)-[4-(thiophen-3-ylethyl)-piperazin-1-yl]-methanon,
(2,3-Dimethyl-1*H*-indol-7-yl)-[4-(2,5-dichlor-thiophen-3-ylethyl)-piperazin-1-yl]-methanon,

(6,7,8,9-Tetrahydro-5*H*-carbazol-3-yl)-(4-phenethyl-piperazin-1-yl)-methanon, Hydrochlorid, F. 235-237°;
(6,7,8,9-Tetrahydro-5*H*-carbazol-3-yl)-[4-(4-fluorphenethyl)-piperazin-1-yl]-methanon,
(6,7,8,9-Tetrahydro-5*H*-carbazol-3-yl)-[4-(thiophen-2-ylethyl)-piperazin-1-yl]-methänon,
(6,7,8,9-Tetrahydro-5*H*-carbazol-3-yl)-[4-(5-chlor-thiophen-2-ylethyl)-piperazin-1-yl]-methanon,
(6,7,8,9-Tetrahydro-5*H*-carbazol-3-yl)-[4-(thiophen-3-ylethyl)-piperazin-1-yl]-methanon,
(6,7,8,9-Tetrahydro-5*H*-carbazol-3-yl)-[4-(2,5-dichlor-thiophen-3-ylethyl)-piperazin-1-yl]-methanon,

(3-Formyl-1*H*-indol-6-yl)-[4-(4-fluorphenethyl)-piperazin-1-yl]-methanon, Hydrochlorid, F. 279,3°;
(1*H*-Indol-6-yl)-[4-(5-chlor-thiophen-2-ylethyl)-piperazin-1-yl]-methanon, Hydrochlorid, F. 257,5-259,0°;
(1*H*-Indol-4-yl)-[4-(5-chlor-thiophen-2-ylethyl)-piperazin-1-yl]-methanon, Hydrochlorid, F. 266-267°;
(3-Cyan-1*H*-indol-5-yl)-[4-(4-fluorphenethyl)-piperazin-1-yl]-methanon, Hydrochlorid, F. 210°;
(3-Cyan-1*H*-indol-7-yl)-[4-(naphth-2-ylethyl)-piperazin-1-yl]-methanon, Hydrochlorid, F. 284,0-285,5°;
(3-Cyan-1*H*-indol-4-yl)-[4-(4-fluorphenethyl)-piperazin-1-yl]-methanon, Hydrochlorid, F. 284,0-285,5°;
(3-Cyan-1*H*-indol-4-yl)-[4-(2-fluorphenethyl)-piperazin-1-yl]-methanon, Hydrochlorid, F. 213-215,5°;
(3-Cyan-1*H*-indol-7-yl)-[4-(2-fluorphenethyl)-piperazin-1-yl]-methanon, Hydrochlorid, F. 212,5-214°;
(3-Aminocarbonyl-1*H*-indol-7-yl)-[4-(4-fluorphenethyl)-piperazin-1-yl]-methanon, Hydrochlorid, F. 280-281°;
(3-Cyan-1*H*-indol-7-yl)-[4-(4-fluorphenethyl)-piperazin-1-yl]-methanon, Methansulfonat, F. 212,5-214°;
(3-Cyan-1*H*-Indol-7-yl)-(4-(5-chlor-thiophen-2-ylethyl)-piperazin-1-yl]-methanon, Hydrochlorid, F. 301,5-303,0°;
(3-Cyan-1*H*-indol-7-yl)-(4-phenethyl-piperazin-1-yl)-methanon, Methansulfonatd, F. 294,7-297°;
(3-Cyan-1*H*-indol-7-yl)-[4-(2,4-difluorphenethyl)-piperazin-1-yl]-methanon, Hydrochlorid, F. 295,6-297,0°;
7-{4-[2-(4-Fluoro-phenyl)-ethyl]-piperazin-1-carbonyl}-1*H*-indoie-2,3-dion.

Die nachfolgenden Beispiele betreffen pharmazeutische Zubereitungen:

### Beispiel A: Injektionsgläser

Eine Lösung von 100 g eines Wirkstoffes der Formel I und 5 g Dinatriumhydrogenphosphat in 3 I zweifach destilliertem Wasser wird mit 2 n Salzsäure auf pH 6,5 eingestellt, steril filtriert, in Injektionsgläser abgefüllt, lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9.38 g NaH₂PO₄ x 2 H₂O, 28.48 g NaH₂PO₄ x 12 H₂O und 0.1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1.2 kg Kartoffelstärke, 0.2 kg Talk und 0.1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird in Ampullen abgefüllt, unter aseptischen Bedingungen lyophilisiert und steril verschlossen. Jede Ampulle enthält 10 mg Wirkstoff.

## Patentansprüche

1. Verbindungen der Formel I 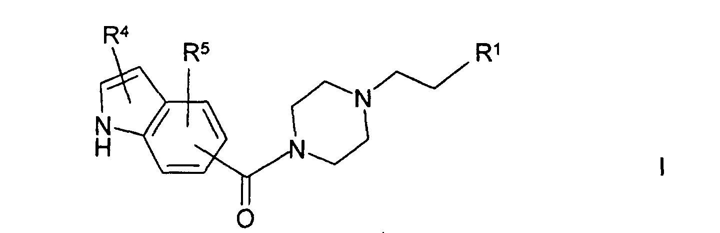 worin
R¹ einen unsubstituierten oder durch R² und/oder R³ substituierten Phenyl- oder Naphthylrest
oder Het¹,
R², R³ jeweils unabhängig voneinander Hal, A, OA, OH oder CN,
R⁴, R⁵ jeweils unabhängig voneinander H, CN, Acyl, Hal, A, OA, OH, CONH₂, CONHA oder CONA₂,
R⁴ und R⁵ zusammen auch Alkylen mit 3-5 C-Atomen,
Het¹ ein- oder zweikerniges unsubstituiertes oder ein- oder zweifach durch Hal, A, OA oder OH substituiertes ungesättigtes heterocyclisches Ringsystem, welches eines, zwei oder drei gleiche oder verschiedene Heteroatome wie Stickstoff, Sauerstoff und Schwefel enthält,
A Alkyl mit 1-6 C-Atomen,
Hal F, Cl, Br oder I,
bedeuten,
und wobei der Indolring auch durch eine Isatineinheit ersetzt sein kann, sowie deren physiologisch unbedenklichen Salze und Solvate,
wobei (1H-Indol-5-yl)-(4-phenethyl-piperazin-1-yl)-methanon ausgeschlossen ist.

2. Verbindungen der Formel I ausgewählt aus einer Gruppe bestehend aus:
(a) (3-Cyan-1*H*-indol-7-yl)-[4-(4-fluorphenethyl)-piperazin-1-yl]-methanon,
(b) (3-Aminocarbonyl-1*H*-indol-7-yl)-[4-(4-fluorphenethyl)-piperazin-1-yl]-methanon
sowie deren physiologisch unbedenklichen Salze und Solvate.

3. Verfahren zur Herstellung von Verbindungen der Formel I gemäß Anspruch 1 oder 2, wobei (1*H*-Indol-5-yl)-(4-phenethyl-piperazin-1-yl)-methanon ausgeschlossen ist, **dadurch gekennzeichnet, daß** man
a) eine Verbindung der Formel II 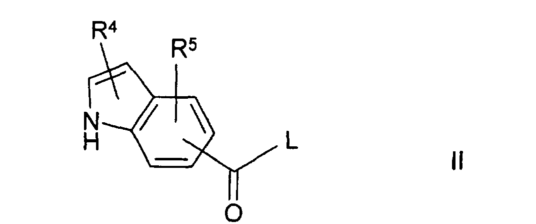 worin L Cl, Br, I oder eine freie oder reaktionsfähig funktionell abgewandelte OH-Gruppe bedeutet,
und R⁴ und R⁵ die in Anspruch 1 angegebenen Bedeutungen haben, mit einer Verbindung der Formel III 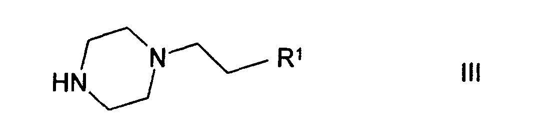 worin R¹ die in Anspruch 1 angegebene Bedeutung hat,
umsetzt,
oder
b) eine Verbindung der Formel IV 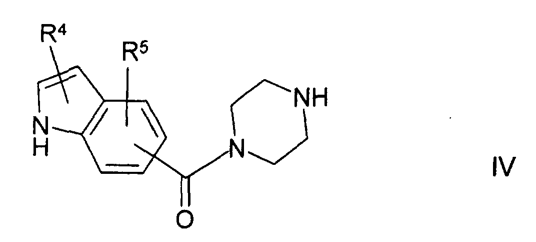 worin R⁴ und R⁵ die in Anspruch 1 angegebenen Bedeutungen haben,
mit einer Verbindung der Formel V
L-CH₂-CH₂-R¹ V
worin L Cl, Br, I oder eine freie oder reaktionsfähig funktionell abgewandelte OH-Gruppe bedeutet, und R¹ die in Anspruch 1 angegebene Bedeutung hat,
umsetzt,
oder
c) gegebenenfalls einen der Reste R¹, R⁴ und/oder R⁵ in einen anderen Rest R¹, R⁴ und/oder R⁵ umwandelt, indem man beispielsweise eine OA-Gruppe unter Bildung einer OH-Gruppe spaltet und/oder eine CHO-Gruppe in eine CN-Gruppe umwandelt
und/oder
eine erhaltene Base der Formel I durch Behandeln mit einer Säure in eines ihrer Salze umwandelt.

4. Verbindungen der Formel I gemäß Anspruch 1 oder 2, sowie ihrer physiologisch unbedenklichen Salze und Solvate, wobei (1*H*-Indol-5-yl)-(4-phenethyl-piperazin-1-yl)-methanon ausgeschlossen ist, als Arzneimittel.

5. Verbindungen der Formel I gemäß Anspruch 1 oder 2, sowie ihrer physiologisch unbedenklichen Salze und Solvate als Arzneimittel mit 5-HT_{2A}-Rezeptor-antagonistischer Wirkung.

6. Arzneimittel nach Anspruch 5 zur Behandlung von Psychosen, Schizophrenie, Depression, neurologischen Störungen, Gedächtnisstörungen, Morbus Parkinson, amyotropher Lateralsklerose, der Alzheimer Krankheit, der Huntington Krankheit, Essstörungen wie Bulimie, Panikattacken, nervöser Anorexie, Schlafstörungen wie auch Schlafapnoe, des prämenstrualen Syndroms, zur Prophylaxe und zur Bekämpfung der Folgen cerebraler Infarktgeschehen wie Schlaganfall und cerebraler Ischämien und/oder zur positiven Beeinflussung von Zwangsverhalten (obsessive-compulsive disorder, OCD).

7. Pharmazeutische Zubereitung, enthaltend mindestens ein Arzneimittel gemäß Anspruch 6, sowie gegebenenfalls Träger- und/oder Hilfsstoffe und gegebenenfalls andere Wirkstoffe.

8. Verwendung von Verbindungen gemäß Anspruch 1 oder 2 und/oder von deren physiologisch unbedenklichen Salzen und Solvaten zur Herstellung eines Arzneimittels mit 5-HT_{2A}-Rezeptor-antagonistischer Wirkung.

9. Verwendung nach Anspruch 8 zur Herstellung eines Arzneimittels zur Behandlung von Psychosen, Schizophrenie, Depression, neurologischen Störungen, Gedächtnisstörungen, Morbus Parkinson, amyotropher Lateralsklerose, der Alzheimer Krankheit, der Huntington Krankheit, Essstörungen wie Bulimie, Panikattacken, nervöser Anorexie, Schlafstörungen wie auch Schlafapnoe, des prämenstrualen Syndroms, zur Prophylaxe und zur Bekämpfung der Folgen cerebraler Infarktgeschehen wie Schlaganfall und cerebraler Ischämien und/oder zur positiven Beeinflussung von Zwangsverhalten (obsessive-compulsive disorder, OCD).

## Claims

1. Compounds of the formula I 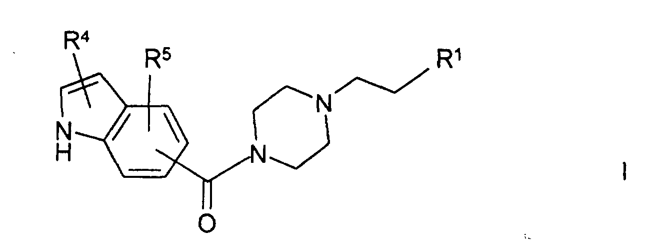 in which
R¹ is a phenyl or naphthyl radical, each of which is unsubstituted or substituted by R² and/or R³,
or Het¹,
R² and R³ are each, independently of one another, Hal, A, OA, OH or CN,
R⁴ and R⁵ are each, independently of one another, H, CN, acyl, Hal, A, OA, OH, CONH₂, CONHA or CONA₂,
R⁴ and R⁵ together are alternatively alkylene having 3-5 carbon atoms,
Het¹ is a monocyclic or bicyclic, unsaturated heterocyclic ring system which is unsubstituted or monosubstituted or disubstituted by Hal, A, OA or OH and which contains one, two or three identical or different heteroatoms, such as nitrogen, oxygen and sulfur,
A is alkyl having 1-6 carbon atoms,
Hal is F, Cl, Br or I,
and where the indole ring may also be replaced by an isatin unit, and physiologically acceptable salts and solvates thereof,
where (1H-indol-5-yl)(4-phenethylpiperazin-1-yl)methanone is excluded.

2. Compounds of the formula I selected from a group consisting of:
(a) (3-cyano-1*H*-indol-7-yl)[4-(4-fluorophenethyl)piperazin-1-yl]-methanone,
(b) (3-aminocarbonyl-1*H*-indol-7-yl)[4-(4-fluorophenethyl)piperazin-1-yl]-methanone,
and the physiologically acceptable salts and solvates thereof.

3. Process for the preparation of compounds of the formula I according to Claim 1 or 2, where (1*H*-indol-5-yl)(4-phenethylpiperazin-1-yl)-methanone is excluded, **characterised in that**
a) a compound of the formula II 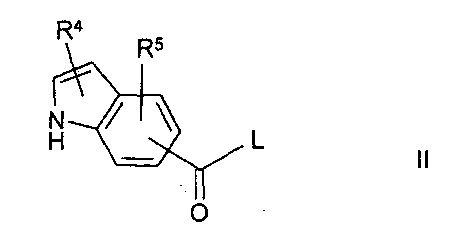 in which L is Cl, Br, I or a free or reactively functionally modified OH group,
and R⁴ and R⁵ are as defined in Claim 1,
is reacted with a compound of the formula III 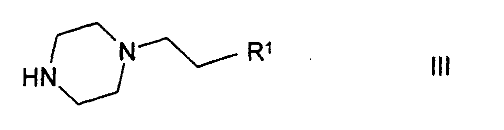 in which R¹ is as defined in Claim 1,
or
b) a compound of the formula IV 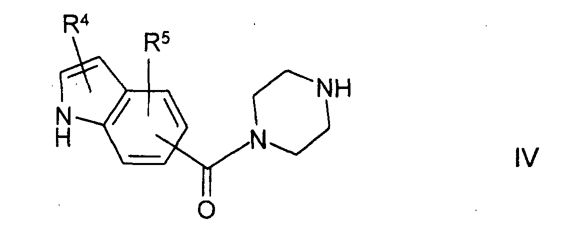 in which R⁴ and R⁵ are as defined in Claim 1,
is reacted with a compound of the formula V
L-CH₂-CH₂-R¹ V
in which L is Cl, Br, I or a free or reactively functionally modified OH group, and R¹ is as defined in Claim 1,
or
c) if desired, one of the radicals R¹, R⁴ and/or R⁵ is converted into another radical R¹, R⁴ and/or R⁵ by, for example, cleaving an OA group with formation of an OH group and/or converting a CHO group into a CN group,
and/or
a resultant base of the formula I is converted into one of its salts by treatment with an acid.

4. Compounds of the formula I according to Claim 1 or 2 and physiologically acceptable salts and solvates thereof, where (1*H*-indol-5-yl)(4-phenethylpiperazin-1-yl)methanone is excluded, as medicaments.

5. Compounds of the formula I according to Claim 1 or 2 and physiologically acceptable salts and solvates thereof as medicaments having a 5-HT_{2A} receptor antagonistic action.

6. Medicament according to Claim 5 for the treatment of psychoses, schizophrenia, depression, neurological disorders, memory disorders, Parkinson's disease, amyotrophic lateral sclerosis, Alzheimer's disease, Huntington's disease, eating disorders, such as bulimia, panic attacks, nervous anorexia, sleep disorders, including sleep apnoea, premenstrual syndrome, for the prophylaxis and combating of the consequences of cerebral infarction, such as strokes and cerebral ischaemia, and/or for positively influencing obsessive-compulsive disorder (OCD).

7. Pharmaceutical preparation comprising at least one medicament according to Claim 6 and, if desired, excipients and/or adjuvants and, if desired, other active ingredients.

8. Use of compounds according to Claim 1 or 2 and/or physiologically acceptable salts and solvates thereof for the preparation of a medicament having a 5-HT_{2A} receptor antagonistic action.

9. Use according to Claim 8 for the preparation of a medicament for the treatment of psychoses, schizophrenia, depression, neurological disorders, memory disorders, Parkinson's disease, amyotrophic lateral sclerosis, Alzheimer's disease, Huntington's disease, eating disorders, such as bulimia, panic attacks, nervous anorexia, sleep disorders, including sleep apnoea, premenstrual syndrome, for the prophylaxis and combating of the consequences of cerebral infarction, such as strokes and cerebral ischaemia, and/or for positively influencing obsessive-compulsive disorder (OCD).

## Revendications

1. Composés répondant à la formule I 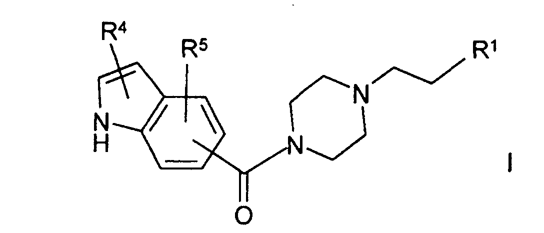 dans laquelle
R¹ représente un radical phényle ou naphtyle, chacun d'entre eux étant non substitué ou substitué par R² et/ou R³,
ou Het¹,
R² et R³ représentent chacun, indépendamment l'un de l'autre, Hal, A, OA, OH ou CN,
R⁴ et R⁵ représentent chacun, indépendamment l'un de l'autre, H, CN, acyle, Hal, A, OA, OH, CONH₂, CONHA ou CONA₂,
R⁴ et R⁵ représentent ensemble de manière alternative alkylène ayant de 3 à 5 atomes de carbone,
Het¹ représente un système de cycle hétérocyclique insaturé monocyclique ou bicyclique, qui est non substitué ou monosubstitué ou disubstitué par Hal, A, OA ou OH et qui contient un, deux ou trois hétéro atomes identiques ou différents, tels que l'azote, l'oxygène et le soufre,
A représente alkyle ayant de 1 à 6 atomes de carbone,
Hal représente F, Cl, Br ou I,
et où le cycle indole peut également être remplacé par un motif isatine, et les sels et les solvats physiologiquement acceptables de ceux-ci, où la (1H-indol-5-yl)(4-phénéthylpipérazin-1-yl)méthanone est exclue.

2. Composés répondant à la formule I choisis parmi un groupe constitué par:
(a) la (3-cyano-1*H*-indol-7-yl)[4-(4-fluorophénéthyl)pipérazin-1-yl]méthanone,
(b) la (3-aminocarbonyl-1*H*-indol-7-yl)[4-(4-fluorophénéthyl)pipérazin-1-yl]méthanone,
et les sels et les solvats physiologiquement acceptables de ceux-ci.

3. Procédé de préparation de composés répondant à la formule I selon la revendication 1 ou 2, où la (1*H*-indol-5-yl)(4-phénéthylpipérazin-1-yl)-méthanone est exclue, **caractérisé en ce que**
a) un composé répondant à la formule II 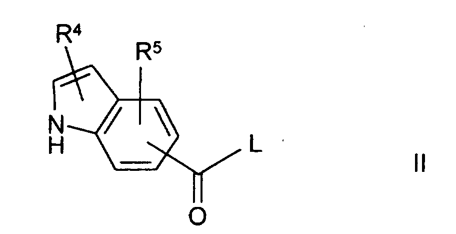 dans laquelle L représente Cl, Br, I ou un groupement OH libre ou modifié de façon réactive et fonctionnelle,
et R⁴ et R⁵ sont tels que définis selon la revendication 1,
est réagi avec un composé répondant à la formule III 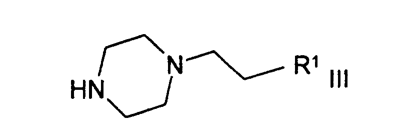 dans laquelle R¹ est tel que défini selon la revendication 1,
ou
b) un composé répondant à la formule IV 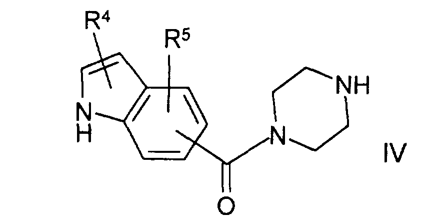 dans laquelle R⁴ et R⁵ sont tels que définis selon la revendication 1, est réagi avec un composé répondant à la formule V
L-CH₂-CH₂-R¹ V
dans laquelle L représente Cl, Br, I ou un groupement OH libre ou modifié de façon réactive et fonctionnelle, et R¹ est tel que défini selon la revendication 1,
ou
c) si on le désire, l'un des radicaux R¹, R⁴ et/ou R⁵ est converti en un autre radical R¹, R⁴ et/ou R⁵, par exemple par le clivage d'un groupement OA avec la formation d'un groupement OH et/ou la conversion d'un groupement CHO en un groupement CN,
et/ou
une base résultante répondant à la formule I est convertie en l'un de ses sels par un traitement par un acide.

4. Composés répondant à la formule I selon la revendication 1 ou 2 et les sels et les solvats physiologiquement acceptables de ceux-ci, où la (1*H*-indol-5-yl)(4-phénéthylpipérazin-1-yl)méthanone est exclue, comme médicaments.

5. Composés répondant à la formule I selon la revendication 1 ou 2 et les sels et les solvats physiologiquement acceptables de ceux-ci, comme médicaments ayant une action antagoniste du récepteur 5-HT_{2A}.

6. Médicament selon la revendication 5 pour le traitement des psychoses, de la schizophrénie, de la dépression, des troubles neurologiques, des troubles de la mémoire, de la maladie de Parkinson, de la sclérose latérale amyotrophique, de la maladie d'Alzheimer, de la maladie de Huntington, des troubles de l'alimentation, tels que la boulimie, des attaques de panique, de l'anorexie nerveuse, des troubles du sommeil, y compris les apnées du sommeil, du syndrôme prémenstruel, pour la prophylaxie et la lutte contre les conséquences de l'infarctus cérébral, telles que les accidents vasculaires cérébraux et l'ischémie cérébrale, et/ou pour influencer positivement le trouble obsessionnel-compulsif (TOC).

7. Préparation pharmaceutique comprenant au moins un médicament selon la revendication 6 et, si on le désire, des excipients et/ou des adjuvants et, si on le désire, d'autres ingrédients actifs.

8. Utilisation de composés selon la revendication 1 ou 2 et/ou de sels et de solvats physiologiquement acceptables de ceux-ci pour la préparation d'un médicament ayant une action antagoniste du récepteur 5-HT_{2A}.

9. Utilisation selon la revendication 8, pour la préparation d'un médicament pour le traitement des psychoses, de la schizophrénie, de la dépression, des troubles neurologiques, des troubles de la mémoire, de la maladie de Parkinson, de la sclérose latérale amyotrophique, de la maladie d'Alzheimer, de la maladie de Huntington, des troubles de l'alimentation, tels que la boulimie, des attaques de panique, de l'anorexie nerveuse, des troubles du sommeil, y compris les apnées du sommeil, du syndrôme prémenstruel, pour la prophylaxie et la lutte contre les conséquences de l'infarctus cérébral, telles que les accidents vasculaires cérébraux et l'ischémie cérébrale, et/ou pour influencer positivement le trouble obsessionnel-compulsif (TOC).
